# EUROPÄISCHE PATENTSCHRIFT

(11) **EP 4 125 743 B1**
(45) Veröffentlichungstag und Bekanntmachung des Hinweises auf die Patenterteilung: **20.12.2023**
(21) Anmeldenummer: 21716160.3
(22) Anmeldetag: 30.03.2021
(51) Int. Cl.: A61F 5/01, A61F 5/058

(54) **DAUMENORTHESE**
THUMB ORTHOSIS
ORTHÈSE DE POUCE

(30) Priorität: 31.03.2020 DE 102020204207
(43) Veröffentlichungstag der Anmeldung: 08.02.2023
(73) Patentinhaber: Bauerfeind AG, 07937 Zeulenroda-Triebes (DE)
(72) Erfinder: BECK, André, 07937 Zeulenroda-Triebes (DE); BAUERFEIND, Hans Bruno, 07937 Zeulenroda-Triebes (DE)
(74) Vertreter: Wohlfahrt, Jan Günther
(86) Internationale Anmeldenummer: PCT/EP2021/058313
(87) Internationale Veröffentlichungsnummer: WO 2021/198266

(56) Entgegenhaltungen:
- DE-A1- 19 511 116
- DE-A1-102016 010 135
- DE-U1-202020 102 689

## Beschreibung

Die vorliegende Erfindung betrifft eine Daumenorthese, insbesondere zur Korrektur von Fehlstellungen des Daumens im Sattelgelenk, wie in den Ansprüchen definiert.

Bei der Rhizarthrose handelt es sich um eine Arthrose des Daumensattelgelenkes. Dabei nutzt sich der Gelenkknorpel ab, es kommt zu einem schmerzhaften Aufeinanderreiben der Gelenkflächen. Typische Symptome sind Schmerzen und Muskelschwäche im Daumen. Das Daumensattelgelenk ist eines der kleinsten, aber am meisten beanspruchten Gelenke des Körpers. Das Sattelgelenk verbindet den ersten Mittelhandknochen und das große Vieleckbein (Os trapezium) der Handwurzelknochen. Wegen seiner besonderen Form verleiht es dem Daumen eine große Beweglichkeit und macht so Greifen und Halten möglich.

Spezielle Handgelenksschienen können das betroffene Gelenk ruhig stellen und entlasten. Die WO 2016/011999 A1 offenbart eine dynamische Orthese, bei der die Knochen des Daumensattelgelenks bei Bewegung auseinandergezogen werden und so die schmerzhafte Reibung vermieden werden soll. Weitere Daumenorthesen sind aus der DE 295 21 749 U1, der WO 2013/160478 A1, der FR 2919491 A1 und der EP 2 453 849 B1 bekannt. DE19511116 beschreibt eine Daumenorthese, zur Korrektur von Fehlstellungen des Daumens im Sattelgelenk, umfassend einen Gurt und einen U-förmig gebogenen Formkörper, der im angelegten Zustand zwischen Daumen und Zeigefinger auf die Hand aufgesetzt ist und den Daumen stützt.

Bei Daumenorthesen aus dem Stand der Technik ist jedoch eine Repositionierung des Mittelhandknochens des Daumens nicht möglich. Vielmehr wird der Daumen durch diese Orthesen vom Zeigefinger weggezogen und in einer Position gegenüber der Handinnenfläche fixiert.

Das der vorliegenden Erfindung zugrunde liegende Problem ist die Bereitstellung einer verbesserten Daumenorthese, insbesondere einer Daumenorthese, die eine gute Repositionierung des Mittelhandknochens des Daumens gegenüber des Handwurzelknochens insbesondere auch dauerhaft erlaubt und durch die der Mittelhandknochen gegenüber dem Handwurzelknochen dauerhaft leicht angehoben wird.

Die vorliegende Erfindung löst das technische Problem durch eine Daumenorthese nach Anspruch 1.

Die vorliegende Erfindung löst das technische Problem durch eine Daumenorthese, insbesondere zur Korrektur von Fehlstellungen des Daumens im Sattelgelenk, umfassend a) einen im wesentlichen formstabilen, U-förmig gebogenen Formkörper, der im angelegten Zustand zwischen Daumen und Zeigefinger auf die Hand aufgesetzt ist und den Daumen stützt und in einem Abstand zum Zeigefinger fixiert und positioniert, wobei der U-förmig gebogene Formkörper einen ersten Endabschnitt und einen zweiten Endabschnitt aufweist, und umfassend b) einen Gurt, der mit einem Gurtende am ersten Endabschnitt des Formkörpers befestigt ist, wobei der zweite Endabschnitt des Formkörpers ein Führungselement zum Führen des Gurts aufweist, wobei der Gurt vom ersten Endabschnitt aus gesehen im angelegten Zustand einmal so um das Handgelenk geführt ist, dass er sich an einem Überlappungspunkt selbst überlappt, wobei die beiden sich überlappenden Gurtabschnitte an dem Überlappungspunkt nicht fest miteinander verbunden sind, und der Gurt am Führungselement des zweiten Endabschnitts geführt wird und das Ende des Gurtes am Gurt reversibel befestigt werden kann.

Das Führungselement dient zum Führen des Gurts, wobei dadurch insbesondere eine Richtungsveränderung des Gurtes, also eine Biegung des Gurtes bis hin zur Richtungsumkehr bewirkt werden kann. Bei dem Überlappungspunkt liegt ein Teilbereich des Gurtes auf einem anderen Teilbereich des Gurtes, wobei sich die Teilbereiche beispielsweise kreuzen können oder parallel übereinander verlaufen können.

Die vorliegende Erfindung löst das technische Problem durch eine Daumenorthese, insbesondere zur Korrektur von Fehlstellungen des Daumens im Sattelgelenk, umfassend a) einen im wesentlichen formstabilen, U-förmig gebogenen Formkörper, der im angelegten Zustand zwischen Daumen und Zeigefinger auf die Hand aufgesetzt ist und den Daumen stützt und in einem Abstand zum Zeigefinger fixiert und positioniert, wobei der U-förmig gebogene Formkörper einen ersten Endabschnitt und einen zweiten Endabschnitt aufweist, und umfassend b) einen Gurt, der mit einem Gurtende am ersten Endabschnitt des Formkörpers befestigt ist, wobei der zweite Endabschnitt des Formkörpers ein Umlenkelement zum Umlenken Gurts aufweist, wobei der Gurt vom ersten Endabschnitt aus gesehen im angelegten Zustand einmal so um das Handgelenk geführt ist, dass er sich an einem Kreuzungspunkt selbst kreuzt, wobei die beiden sich kreuzenden Gurtabschnitte an dem Kreuzungspunkt nicht fest miteinander verbunden sind, und der Gurt am Umlenkelement des zweiten Endabschnitts umgelenkt wird und das Ende des Gurtes am Gurt reversibel befestigt werden kann.

Das Führungselement dient also insbesondere zu einer Richtungsveränderung des Gurtes. Das Führungselement ist also insbesondere als Richtungsveränderungselement ausgestaltet.

Insbesondere kann vorgesehen sein, dass es sich bei der durch das Führungselement herbeigeführten Richtungsveränderung um eine Krümmung des Gurtverlaufs handelt.

In einer bevorzugten Ausführungsform handelt es sich also bei dem Führungselement zum Führen des Gurts um ein Umlenkelement zum Umlenken des Gurts. Die Richtungsveränderung des Gurtes ist also eine Richtungsumkehr. In einer bevorzugten Ausführungsform handelt es sich also bei dem Überlappungspunkt um einen Kreuzungspunkt und bei den beiden sich überlappenden Gurtabschnitten um zwei sich kreuzenden Gurtabschnitte.

Die erfindungsgemäße Daumenorthese führt in vorteilhafter Weise zu einer Repositionierung des Daumens, bei der der Daumen in die Ebene der Handfläche gezogen wird. Je mehr Spannung in den Gurt eingetragen wird, umso weiter positioniert sich der Daumen in vorteilhafter Weise in Richtung Handflächenebene, was überraschender Weise zu einer schmerzlindernden Positionierung des Daumens führt.

Die erfindungsgemäße Ausführungsform der sich überlappenden, insbesondere kreuzenden Gurtabschnitte, bei der die beiden sich überlappenden, insbesondere kreuzenden Gurtabschnitte an dem Überlappungspunkt, insbesondere Kreuzungspunkt nicht fest miteinander verbunden sind, führt zu einem vorteilhaften dynamischen Überlappungspunkt, insbesondere Kreuzungspunkt. Damit ist die Position des Überlappungspunkts, insbesondere Kreuzungspunkts nicht vorgegeben und fest, sondern dynamisch und kann sich an die entsprechende Hand- und Daumengröße anpassen. Somit ist die gewünschte Repositionierung des Daumens, insbesondere in eine Position in die Ebene der Handfläche und das Anheben des Mittelhandknochens gegenüber dem Handwurzelknochen in vorteilhafter Weise möglich. Bei einem dynamischen Überlappungspunkt können sich die beiden sich überlappenden Gurtbereiche in vorteilhafter Weise gegeneinander verschieben.

Im Zusammenhang mit der vorliegenden Erfindung ist für den Fachmann ersichtlich unter einem Kreuzungspunkt die Fläche verstanden, an der sich beide kreuzenden Gurtabschnitte überlappen. Somit kann der Kreuzungspunkt auch als flächiger Kreuzungspunkt oder Kreuzungsfläche verstanden werden.

In einer bevorzugten Ausführungsform liegt der Überlappungspunkt, insbesondere Kreuzungspunkt in einem definierten Überlappungsbereich, insbesondere Kreuzungsbereich.

In einer bevorzugten Ausführungsform ist der Überlappungspunkt, insbesondere Kreuzungspunkt auf dem Gurt so positioniert, dass er etwa 1,5 bis 3 cm vom am ersten Endabschnitt des Formkörpers befestigten Gurtende entfernt ist.

In einer bevorzugten Ausführungsform ist der Überlappungsbereich, insbesondere Kreuzungsbereich des Gurts so ausgestaltet, dass die beiden sich überlappenden bzw. kreuzenden Gurtabschnitte gegeneinander verrutschbar gelagert sind. Der Überlappungsbereich, insbesondere Kreuzungsbereich kann insbesondere so ausgestaltet sein, dass die beiden sich überlappenden bzw. kreuzenden Gurtabschnitte nur in dem Überlappungsbereich, insbesondere Kreuzungsbereich gegeneinander verrutschen können.

In einer bevorzugten Ausführungsform ist der Überlappungsbereich, insbesondere Kreuzungsbereich des Gurts so ausgestaltet, dass einer der beiden sich überlappenden, insbesondere kreuzenden Gurtabschnitte eine Führung für den anderen der beiden sich überlappenden, insbesondere kreuzenden Gurtabschnitte aufweist. Bevorzugt ist die Führung so ausgestaltet, dass der geführte Gurtabschnitt längs und quer zu dem die Führung aufweisende Gurtabschnitt verrutschbar gelagert ist.

Eine solche Führung erlaubt in vorteilhafter Weise die erfindungsgemäße Flexibilität des Überlappungspunkts, insbesondere Kreuzungspunkts der beiden Gurtabschnitte, sorgt aber gleichzeitig dafür, dass der Überlappungspunkt, insbesondere Kreuzungspunkt, nur soweit verschiebbar ist, dass die gewünschte Repositionierung auftritt. Somit wird ein unerwünschtes Verrutschen des Gurts beim Tragen der Daumenorthese vermieden.

In einer bevorzugten Ausführungsform ist der Überlappungsbereich, insbesondere Kreuzungsbereich als Lasche an oder im Gurt ausgebildet, durch die der Gurt hindurchgezogen werden kann, wobei die Lasche in dem Bereich des Gurtes angeordnet ist, der dem ersten Endabschnitt des Formkörpers folgt. Eine Lasche als Führung ist dünn und erlaubt in einfacher Weise das verrutschen des geführten Gurtabschnitts.

In einer bevorzugten Ausführungsform bildet die Lasche einen Schlitz, der breiter ist als die Breite des Gurtes.

Bevorzugt hat die Führung, insbesondere die Lasche also eine Länge, die größer ist als die Breite des Gurts im geführten Gurtabschnitt, so dass der geführte Gurtabschnitt in der Lasche verrutschen kann. Bevorzugt hat die Führung, insbesondere die Lasche eine Länge von mindestens dem 1,2-fachen der Breite des geführten Gurtabschnitts, besonders bevorzugt von mindestens dem 1,5-fachen der Breite des geführten Gurtabschnitts. Bevorzugt hat die Führung, insbesondere die Lasche eine Länge von höchstens dem 3-fachen der Breite des geführten Gurtabschnitts, besonders bevorzugt von höchstens dem 2-fachen der Breite des geführten Gurtabschnitts. Bevorzugt hat die Führung, insbesondere die Lasche eine Länge von mindestens dem 1,2-fachen und höchstens dem 3-fachen der Breite des geführten Gurtabschnitts, besonders bevorzugt von mindestens dem 1,5-fachen und höchstens dem 2-fachen der Breite des geführten Gurtabschnitts.

Der unter der Lasche des Gurtes liegende Grundgurt ist bevorzugt als Vollmaterial ausgebildet, damit sich auf dem abgedeckten Gewebe kein Fensterödem bilden kann. Bevorzugt ist daher der Grundgurt an dieser Stelle gleichförmig durchgehend und die Lasche auf diesen aufgesetzt oder an diesen angesetzt und nicht als Einschnitt des Grundgurts ausgearbeitet.

In einer alternativen Ausführungsform ist der Überlappungsbereich, insbesondere Kreuzungsbereich als Klettverschluss ausgebildet. Auch dieser erlaubt eine dynamische Positionierung des Überlappungspunkts, insbesondere Kreuzungspunkts.

In einer bevorzugten Ausführungsform kann das Ende des Gurtes am Gurt über einen Dornverschluss, über einen Klettverschluss oder über einen Haken/Ösen-Verschluss reversibel befestigt werden. In einer bevorzugten Ausführungsform weist das Ende des Gurtes am Gurt einen Dornverschluss, einen Klettverschluss oder einen Haken/Ösen-Verschluss auf, mit dem es am Gurt reversibel befestigt werden kann. In einer bevorzugten Ausführungsform weist das Ende des Gurtes am Gurt einen Dorn, eine Klett- oder Flauschfläche oder einen Haken oder eine Öse auf, womit es am Gurt reversibel befestigt werden kann.

In einer bevorzugten Ausführungsform sind der Formkörper und der Gurt einstückig ausgebildet.

In einer bevorzugten Ausführungsform ist das Führungselement am zweiten Endabschnitt des Formkörpers als Lasche ausgebildet oder als mindestens ein Einschnitt in dem zweiten Endabschnitt ausgebildet oder als Lochband ausgebildet.

In einer bevorzugten Ausführungsform ist das Führungselement am zweiten Endabschnitt des Formkörpers als Lasche oder als zwei Einschnitte in dem zweiten Endabschnitt ausgebildet

In einer bevorzugten Ausführungsform ist das Umlenkelement am zweiten Endabschnitt des Formkörpers als Einschnitt in den zweiten Endabschnitt ausgebildet.

In einer bevorzugten Ausführungsform kann das Führungselement an den Gurt über ein Fixierungselement befestigt werden. In einer bevorzugten Ausführungsform kann das Führungselement an die Löcher des Gurts über ein Fixierungselement befestigt werden.

In einer bevorzugten Ausführungsform kann das Führungselement an den Gurt über ein Fixierungselement reversibel befestigt werden. In einer bevorzugten Ausführungsform kann das Führungselement an die Löcher des Gurts über ein Fixierungselement reversibel befestigt werden.

In einer bevorzugten Ausführungsform kann das Führungselement an den Gurt über einen Dornverschluss, über einen Klettverschluss oder über einen Haken/Ösen-Verschluss reversibel befestigt werden.

In einer bevorzugten Ausführungsform ist das Führungselement als Lochband ausgebildet, dem bevorzugt ein Fixierungselement, zum Beispiel ein Dornverschluss, zum Fixieren mindestens eines der Löcher des Lochbands an den Gurt, insbesondere an mindestens eins der Löcher des Gurts, zugeordnet ist. In einer bevorzugten Ausführungsform ist das Führungselement also als Lochband und Fixierungselement ausgebildet. Das Fixierungselement, beispielsweise ein Dornverschluss kann eines der Löcher des Führungselements mit einem der Löcher des Gurts verbinden, sodass der Richtungsverlauf des Gurts je nach gewähltem Loch des Führungselements in Kombination mit dem gewählten Loch des Gurts verschieden geführt werden kann. Diese Ausführungsform ermöglicht in vorteilhafter Weise eine spezifischere Anpassung der gewünschten Führung des Gurts.

In einer bevorzugten Ausführungsform ist der Formkörper sattelförmig.

In einer bevorzugten Ausführungsform weist der Formkörper Luftlöcher auf. Dies erlaubt eine Belüftung und eine Feuchtigkeitsabfuhr im durch den Formkörper abgedeckten Sattelbereich.

In einer bevorzugten Ausführungsform besteht der Formkörper aus einem anformbaren Material. Damit kann der Formkörper, wenn gewünscht oder notwendig an die genaue Anatomie der Hand des Patienten angepasst werden.

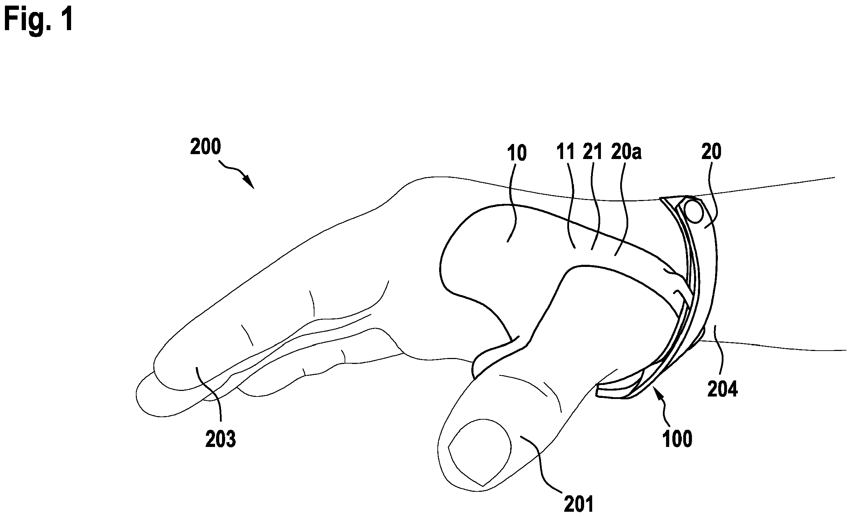

In einer besonders bevorzugten Ausführungsform ist die Daumenorthese ausgebildet mit den in den Figuren 1,3,4 und 6 dargestellten Merkmalen, insbesondere der Ausgestaltung der dort gezeigten Lasche. Bevorzugt ist die Lasche am Gurt nicht wie eine Lasche zur Fixierung eines Armband-Verschlusses ausgebildet, also quer zum Gurt, sondern längs zum Gurt, wodurch insbesondere ein Kreuzungsbereich ausgebildet werden kann.

Bevorzugt verläuft die Lasche in etwa längs zum Gurt. Bevorzugt verläuft die Lasche am Gurt so, dass ein Kreuzungsbereich ausgebildet werden kann. Bevorzugt verläuft die Lasche längs zum Gurt. Bevorzugt verläuft die Lasche nicht quer zum Gurt.

Weitere bevorzugte Ausführungsformen ergeben sich aus den Unteransprüchen.

Die Erfindung wird anhand des Ausführungsbeispiels der Figuren 1 bis 14 näher erläutert, ohne dass dieses Ausführungsbeispiel einschränkend zu verstehen ist.

Es zeigen:
- Figur 1: eine erste Ausführungsform der erfindungsgemäßen Daumenorthese an einer Hand;
- Figur 2: die Daumenorthese aus Figur 1 aus einer anderen Perspektive;
- Figur 3: die Daumenorthese aus Figur 1 aus einer anderen Perspektive;
- Figur 4: die Daumenorthese aus Figur 1 in nicht angelegtem Zustand;
- Figur 5: die Daumenorthese aus Figur 4 aus einer anderen Perspektive;
- Figur 6: die Daumenorthese aus Figur 4 mit gekreuztem Gurt;
- Figur 7: die Daumenorthese aus Figur 6 aus einer anderen Perspektive;
- Figur 8: eine zweite Ausführungsform der erfindungsgemäßen Daumenorthese an einer Hand;
- Figur 9: die Daumenorthese aus Figur 8 aus einer anderen Perspektive;
- Figur 10: die Daumenorthese aus Figur 8 aus einer anderen Perspektive;
- Figur 11: die Daumenorthese aus Figur 8 in nicht angelegtem Zustand;
- Figur 12: die Daumenorthese aus Figur 11 aus einer anderen Perspektive;
- Figur 13: die Daumenorthese aus Figur 11 mit überlappendem Gurt;
- Figur 14: die Daumenorthese aus Figur 13 aus einer anderen Perspektive;
- Figur 15: eine weitere Ausführungsform des Führungselements einer erfindungsgemäßen Daumenorthese.

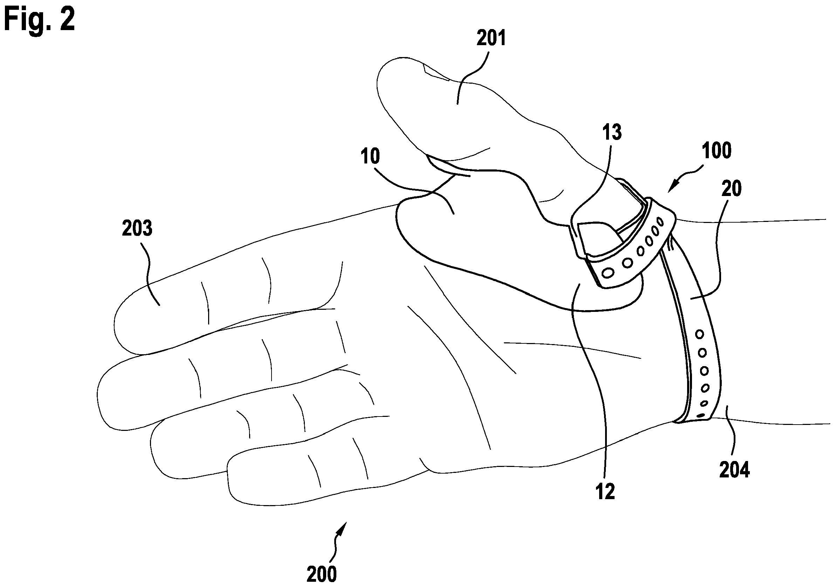

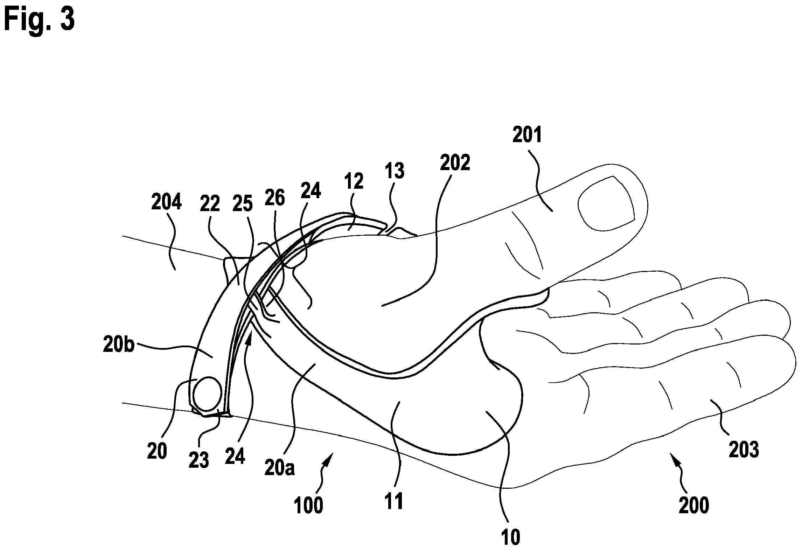

Figur 1 zeigt eine bevorzugte ausführungsform einer erfindungsgemäßen Daumenorthese (100) an einer rechten Hand (200) eines Patienten. Der Fachmann kann ohne weiteres eine entsprechende Daumenorthese für eine linke Hand herstellen. Die Daumenorthese (100) weist einen U-förmig gebogenen Formkörper (10) auf, der sattelförmig zwischen Daumen (201) und Zeigefinger (203) auf der Hand (200) aufliegt. Der U-förmig gebogene Formkörper (10) weist einen ersten Endabschnitt (11) auf, der im angezogenen Zustand auf der Außenfläche der Hand (200) liegt. An diesem ersten Endabschnitt (11) ist ein Gurt (20) mit seinem ersten Gurtende (21) des ersten Gurtabschnitts (20a) befestigt. Der Gurt (20) verläuft sich kreuzend um das Handgelenk (204). Der genaue Verlauf des Gurtes (20) ist in den Figuren 2 und 3 zu erkennen.

Figur 2 zeigt die Daumenorthese (100) aus Figur 1 aus einer anderen Perspektive. Zu sehen ist wieder die Hand (200), bei der zwischen Daumen (201) und Zeigefinger (203) der U-förmig gebogene Formkörper (10) aufliegt. Dieser weist an der Handinnenfläche einen zweiten Endabschnitt (12) mit einem als Schlitz ausgebildeten Umlenkelement (13) auf. In dem Umlenkelement (13) wird der sich kreuzend um das Handgelenk (204) verlaufende Gurt (20) umgelenkt.

Figur 3 zeigt die Daumenorthese (100) aus Figur 1 aus einer dritten Perspektive. Zu sehen ist wieder die Hand (200), bei der zwischen Daumen (201) und Zeigefinger (203) der U-förmig gebogenen Formkörper (10) aufliegt. Am ersten Endabschnitt (11) beginnt der Gurt (20) um das Handgelenk (204) zu verlaufen. Im ersten Gurtabschnitt (20a) weist er einen als Lasche (25) ausgebildeten Kreuzungsbereich (24) auf. Der Gurt (20) verläuft um das Handgelenk (204) und kreuzt sich im Kreuzungsbereich (24), indem er durch den durch die Lasche (25) gebildeten Schlitz (26) hindurchgeführt ist, so dass sich ein Kreuzungspunkt (22) ergibt. Der Gurt (20) verläuft dann zum zweiten Endabschnitt (12) des Formkörpers (10) und wird dort am Umlenkelement (13) umgelenkt. Zur Straffung des Gurtes (20) liegt sein zweiter Gurtabschnitt (20b) bis zu seinem zweiten Gurtende (23) am ersten Gurtabschnitt (20a) an und ist reversibel mit diesem über einen nicht sichtbaren Dornverschluss verbunden.

Der Kreuzungspunkt (22) liegt dabei zwischen Radiusköpfchen und Daumensattelgelenk (202) der Hand (200).

Die in den Figuren 1 bis 3 gezeigte Daumenorthese (100) führt in vorteilhafter Weise dazu, dass dadurch dass der Gurt (20) sich im Kreuzungsbereich (24) dynamisch kreuzt, eine Repositionierung des Daumens (201) erfolgt, bei der der Daumen (201), wie insbesondere in Figur 2 sichtbar, nicht der Handinnenfläche gegenüber liegt, sondern in der Ebene der restlichen Hand (200) liegt. Darüber hinaus wird der Mittelhandknochen in vorteilhafter Weise gegenüber dem Handwurzelknochen leicht angehoben. Der Daumen (201) wird also durch ein Festziehen des Gurts (20) nach dorsal gezogen und gleichzeitig abduziert. Gleichzeitig fixiert der sich kreuzende Gurt (20) die Daumenorthese (100) am Handgelenk. Es muss in vorteilhafter Weise für die volle Funktion der Daumenorthese (100) nur der eine Gurt (20) angezogen werden.

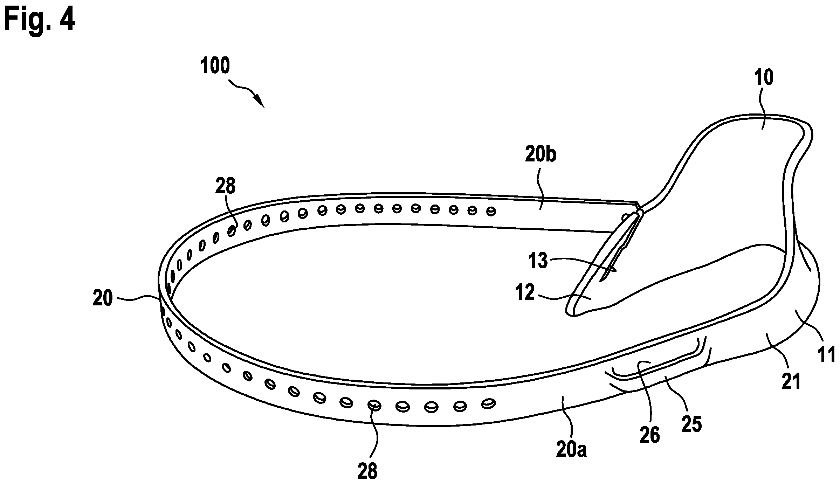

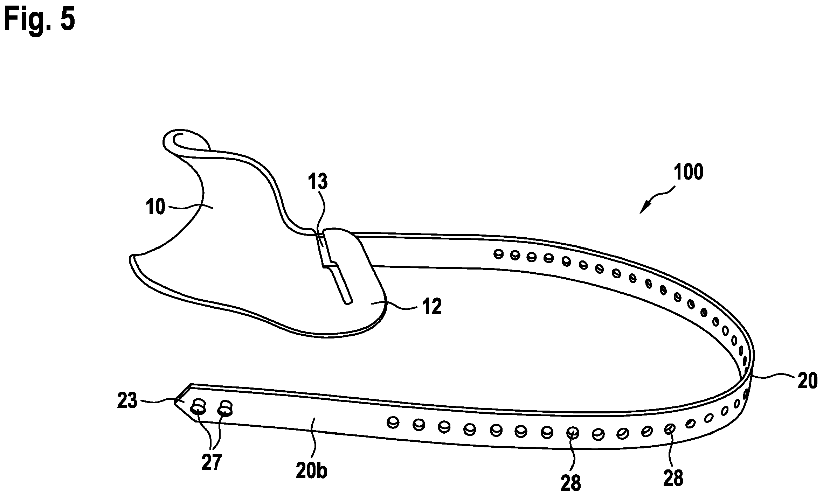

Die Figuren 4 und 5 zeigen die Daumenorthese (100) ohne Hand. Zu sehen ist der U-förmig beziehungsweise sattelförmig gebogenen Formkörper (10) mit dem ersten Endabschnitt (11) und dem zweiten Endabschnitt (12). Am ersten Endabschnitt (11) ist der erste Gurtabschnitt (20a) des Gurts (20) mit seinem ersten Gurtende (21) befestigt. Der erste Gurtabschnitt (20a) weist eine Lasche (25) auf, durch deren Schlitz (26), der breiter als der Gurt (20) ist, der Gurt (20) dynamisch und beweglich hindurchgeführt werden kann. Die Lasche (25) verläuft längs zum Gurt (20) und nicht quer wie eine gewöhnliche Lasche zum Fixieren eines Gurts wie eines Uhrarmbands verlaufen würde. Der Gurt (20) weist Löcher (28) eines Dornverschlusses auf, in die zwei Dornen (27) auf dem zweiten Gurtabschnitt (20b) am zweiten Gurtende (23) zum Schließen des Gurtes (20) hineingesteckt werden können, nachdem der Gurt (20) durch das als Schlitz ausgebildete Umlenkelement (13) des zweiten Endabschnitts (12) des Formkörpers (10) umgelenkt wurde und damit festgezogen werden kann.

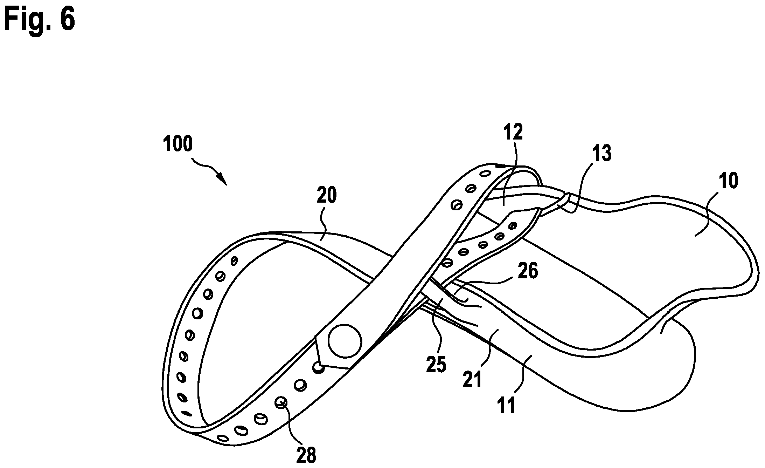

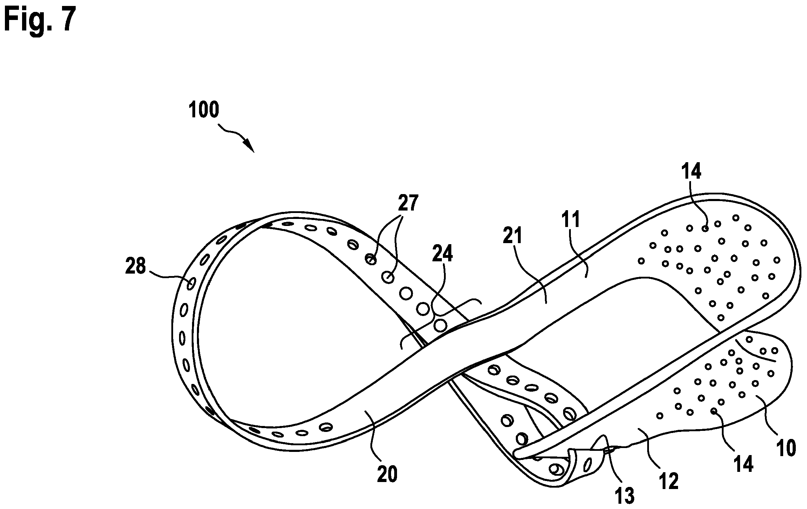

Die Figuren 6 und 7 zeigen die Daumenorthese (100) aus den Figuren 4 und 5 mit dem gekreuzten Verlauf des Gurts (20). Der Gurt (20) beginnt am ersten Gurtende (21) am ersten Endabschnitt (11) des U-förmigen Formkörpers (10) und bildet eine Schlinge. Dann kreuzt sich der Gurt (20) selber in dem Kreuzungsbereich (24), indem er durch den Schlitz (26) der Lasche (25) hindurchgeführt ist. Der Gurt (20) wird dann am zweiten Endabschnitt (12) des U-förmigen Formkörpers (10) durch das Umlenkelement (13) umgelenkt und über den aus Dornen (27) und Löchern (28) bestehenden Dornenverschluss mit sich selbst zugfest und wieder lösbar befestigt. Durch die Vielzahl der Löcher (28) ist dabei eine an die Handgelenksdicke angepasste Befestigung möglich.

In Figur 7 sind darüber hinaus noch bevorzugte Luftlöcher (14) in dem Formkörper (10) zu sehen, die eine Belüftung der unter dem Formkörper (10) liegenden Handfläche erlauben.

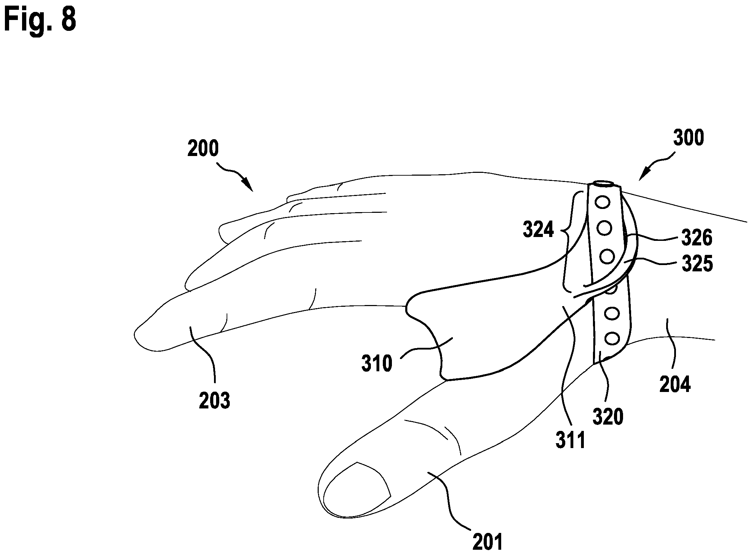

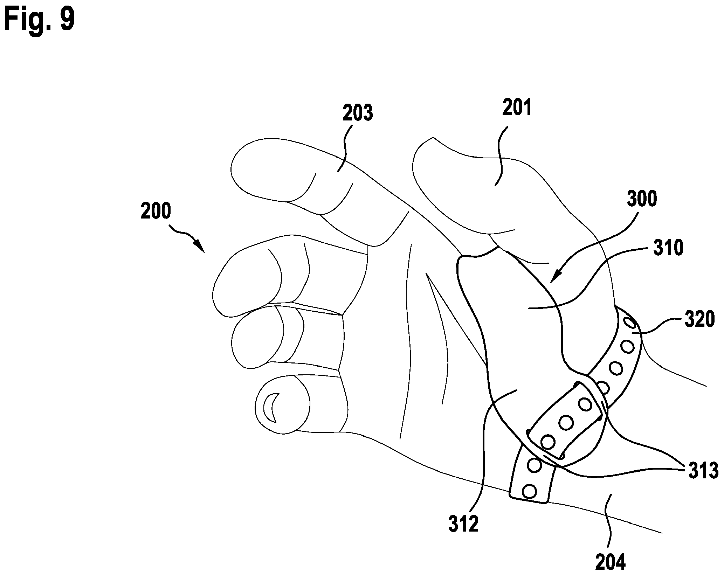

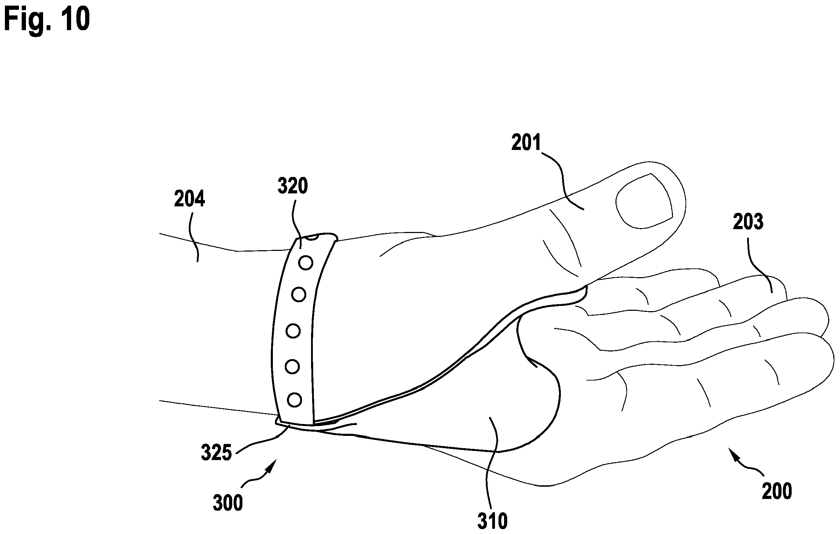

Figur 8 zeigt eine alternative Ausführungsform (300) einer erfindungsgemäßen Daumenorthese, hier wieder an einer rechten Hand (200) mit Daumen (201), Zeigefinger (203) und Handgelenk (204). Auch für diese Ausführungsform der Daumenorthese (300) kann der Fachmann ohne weiteres eine entsprechende Daumenorthese für eine linke Hand bereitstellen. Die Daumenorthese (300) weist einen U-förmig gebogenen Formkörper (310) auf, der sattelförmig zwischen Daumen (201) und Zeigefinger (203) auf der Hand (200) aufliegt. Der U-förmig gebogene Formkörper (310) weist einen ersten Endabschnitt (311) auf, der im angezogenen Zustand auf der Außenfläche der Hand (200) liegt. An diesem ersten Endabschnitt (311) ist ein Gurt (320) mit seinem hier nicht sichtbaren ersten Gurtende des ersten Gurtabschnitts befestigt. Der Gurt (320) verläuft teilweise überlappend um das Handgelenk (204). Dabei wird durch eine Lasche (325) gebildeten Schlitz (326) als Führungselement der Gurt (320) geführt, sodass sich ein Überlappungsbereich beziehungsweise Überlappungspunkt (324) ergibt. Der genaue Verlauf des Gurtes (320) ist in den Figuren 9 und 10 zu sehen.

Figur 9 zeigt die Daumenorthese (300) aus Figur 8 aus einer anderen Perspektive. Zu sehen ist wieder die Hand (200), bei der zwischen Daumen (201) und Zeigefinger (203) der U-förmig gebogene Formkörper (310) aufliegt. Dieser weist an der Handinnenfläche einen zweiten Endabschnitt (312) mit einem als zwei Laschen ausgebildeten Führungselement (313) auf. In dem Führungselement (313) wird der sich überlappend um das Handgelenk (204) verlaufende Gurt (320) umgelenkt.

Figur 10 zeigt die Daumenorthese (300) aus Figur 8 aus einer dritten Perspektive. Zu sehen ist wieder die Hand (200), bei der zwischen Daumen (201) und Zeigefinger (203) der U-förmig gebogene Formkörper (310) anliegt. Im Bereich des Handgelenks (204) wird der Gurt (320) durch eine Lasche (325) geführt, wodurch der Gurt (320) einen Überlappungsbereich bildet.

Auch die in der Ausführungsform der Figuren 8 bis 10 gezeigte Daumenorthese (300) führt in vorteilhafter Weise dazu, dass dadurch, dass der Gurt (320) sich im Überlappungsbereich (324) dynamisch überlappt, eine Repositionierung des Daumens (201) erfolgt, bei der der Daumen (201), wie insbesondere in Figur 9 sichtbar, nicht der Handinnenfläche gegenüberliegt, sondern in der Ebene der restlichen Hand (200). Darüber hinaus wird auch hier der Mittelhandknochen in vorteilhafter Weise gegenüber dem Handwurzelknochen leicht angehoben. Der Daumen (201) wird also durch ein Festziehen des Gurtes (320) ebenfalls nach dorsal gezogen und gleichzeitig abduziert. Gleichzeitig fixiert der sich überlappende Gurt (320) die Daumenorthese (300) am Handgelenk (204). Es muss auch hier in vorteilhafter Weise für die volle Funktion der Daumenorthese (300) nur der eine Gurt (320) angezogen werden, wobei die Handhabung in dieser Ausführungsform noch leichter ist als bei der alternativen Ausführungsform gemäß der Figuren 1 bis 7.

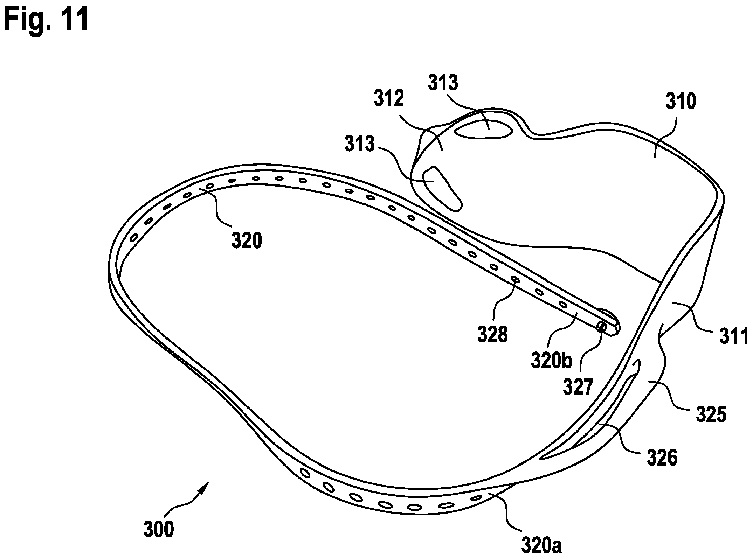

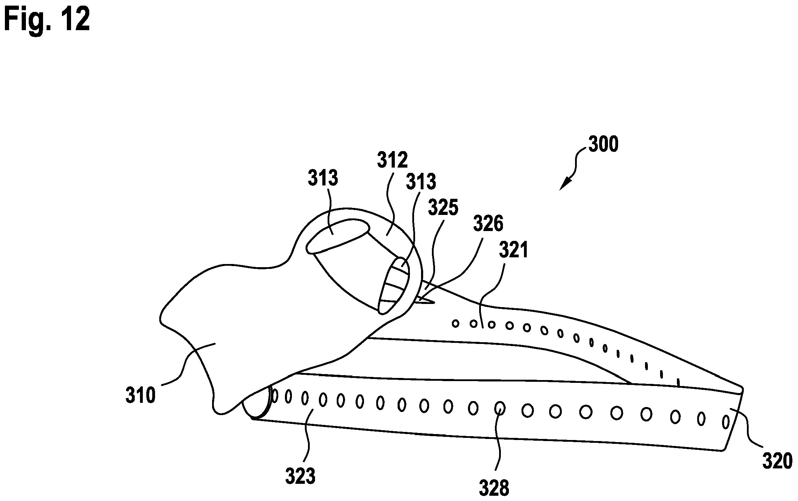

Die Figuren 11 und 12 zeigen die Daumenorthese (300) ohne Hand. Zu sehen ist der U-förmig beziehungsweise sattelförmig gebogene Formkörper (310) mit dem ersten Endabschnitt (311) und dem zweiten Endabschnitt (312). Am ersten Endabschnitt (311) ist der erste Gurtabschnitt (320a) des Gurts (320) mit seinem ersten Gurtende (321) befestigt. Der erste Gurtabschnitt (320a) weist eine Lasche (325) auf, durch deren Schlitz (326), der breiter als der Gurt (320) ist, der Gurt (320) dynamisch und beweglich hindurchgeführt werden kann. Der Gurt (320) weist Löcher (328) eines Dornverschlusses auf, in die ein Dorn (327) auf dem zweiten Gurtabschnitt (320b) am zweiten Gurtende (323) zum Schließen des Gurts (320) hineingesteckt werden können, nachdem der Gurt (320) durch das als zwei Laschen mit Schlitze ausgebildete Führungselement (313) des zweiten Endabschnitts (312) des Formkörpers (310) geführt wurde und damit festgezogen werden kann.

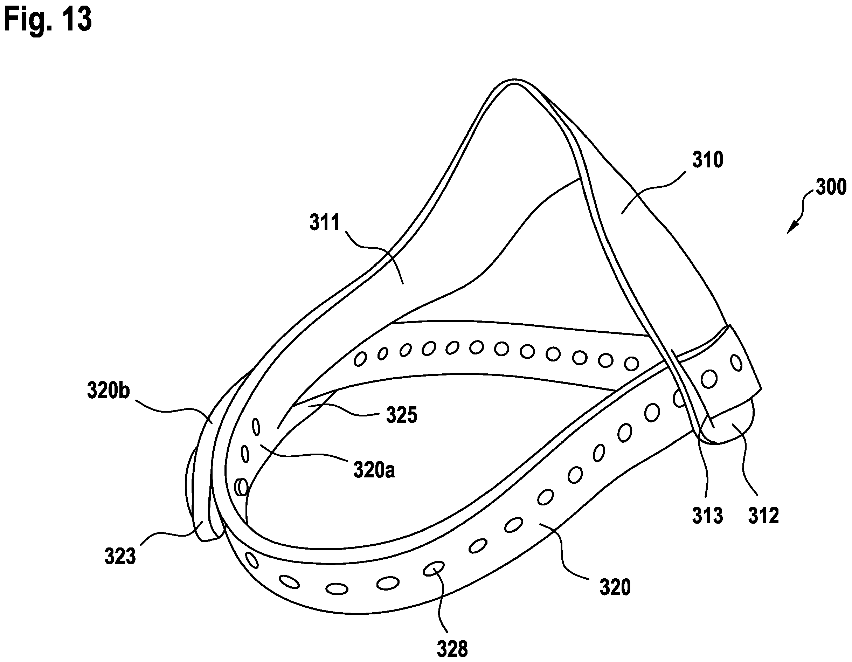

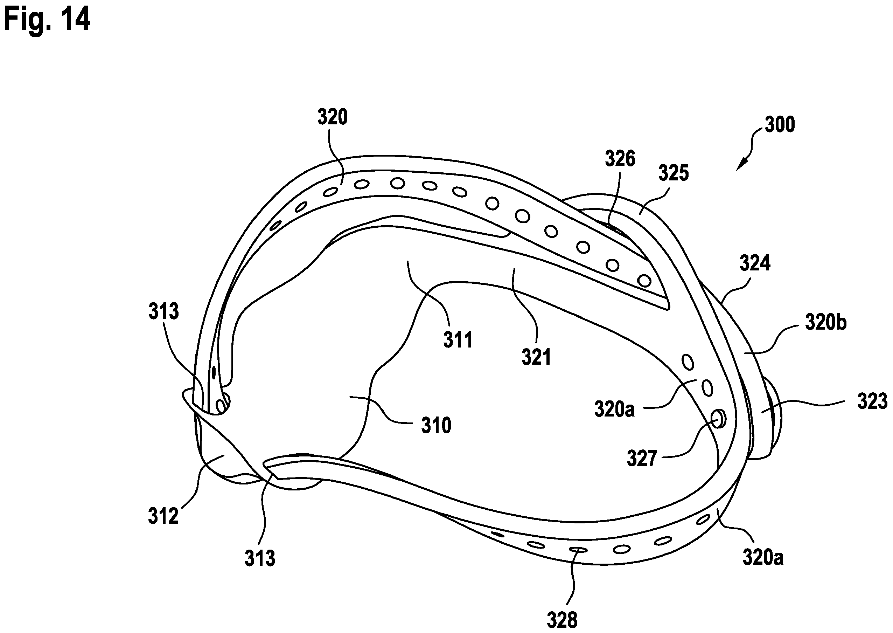

Die Figuren 13 und 14 zeigen die Daumenorthese (300) aus den Figuren 11 und 12 mit dem überlappenden Verlauf (324) des Gurts (320). Der Gurt (320) beginnt am ersten Gurtende (321) am ersten Endabschnitt (311) des U-förmigen Formkörpers (310) und bildet eine Schlinge. Dann überlappt der Gurt (320) mit sich selber in dem Überlappungsbereich (324), in dem er durch den Schlitz (326) der Lasche (325) hindurchgeführt ist. Der Gurt (320) wird am zweiten Endabschnitt (312) des U-förmigen Formkörpers (310) durch das Führungselement (313) geführt und über den aus einem Dorn (327) und Löchern (328) bestehenden Dornverschluss im Überlappungsbereich (324) mit sich selbst zugfest und wieder lösbar befestigt. Durch die Vielzahl der Löcher (328) ist dabei eine an der Handgelenksdicke angepasste Befestigung möglich. Der Dorn (327) liegt dabei im zweiten Gurtabschnitt (320b), also im Bereich des zweiten Gurtendes (323). Der erste Gurtabschnitt (320a) überlappt hier also mit dem zweiten Gurtabschnitt (320b).

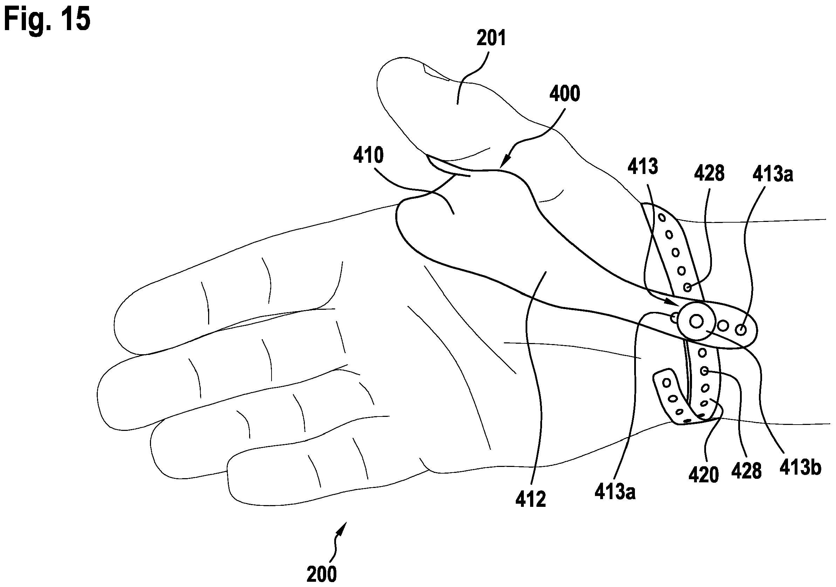

Figur 15 zeigt eine weitere Daumenorthese (400) an einer rechten Hand (200) mit Daumen (201), bei der dem Formkörper (410) mit dem zweiten Endabschnitt (412) eine alternative Ausführungsform des Führungselements (413) folgt. Das Führungselement (413) besteht hier aus einem Lochband mit Löchern (413a), denen ein Dornverschluss (413b) zugeordnet ist. Der Dornverschluss (413b) kann eines der Löcher (413a) des Führungselements (413) mit einem der Löcher (428) des Gurts (420) reversibel verbinden, sodass der Richtungsverlauf des Gurts (420) je nach gewähltem Loch (413a) des Führungselements (413) in Kombination mit dem gewählten Loch (428) des Gurts (420) verschieden geführt werden kann. Diese Ausführungsform ermöglicht in vorteilhafter Weise eine spezifischere Anpassung der gewünschten Führung des Gurts (420).

### Bezugszeichenliste:

- 10: U-förmig gebogenen Formkörper
- 11: erster Endabschnitt
- 12: zweiter Endabschnitt
- 13: Umlenkelement
- 14: Luftlöcher
- 20: Gurt
- 20a: erster Gurtabschnitt
- 20b: zweiter Gurtabschnitt
- 21: erstes Gurtende
- 22: Kreuzungspunkt
- 23: zweites Gurtende
- 24: Kreuzungsbereich
- 25: Lasche im Kreuzungsbereich
- 26: Schlitz
- 27: Dorn eines Dornverschlusses
- 28: Loch eines Dornverschlusses
- 100: Daumenorthese
- 200: Hand
- 201: Daumen
- 202: Sattelgelenk
- 203: Zeigefinger
- 204: Handgelenk
- 300: Daumenorthese
- 310: Formkörper
- 311: erster Endabschnitt
- 312: zweiter Endabschnitt
- 313: Führungselement
- 320: Gurt
- 320a: erster Gurtabschnitt
- 320b: zweiter Gurtabschnitt
- 321: erstes Gurtende
- 323: zweites Gurtenede
- 324: Überlappungsbereich oder Überlappungspunkt
- 325: Lasche im Überlappungsbereich
- 326: Schlitz
- 327: Dorn
- 328: Löcher
- 400: Daumenorthese
- 410: Formkörper
- 412: zweiter Endabschnitt
- 413: Führungselement
- 413a: Löcher des Führungselements
- 413b: Dornverschluss
- 420: Gurt
- 428: Löcher des Gurts

## Patentansprüche

1. Daumenorthese (100, 300, 400), insbesondere zur Korrektur von Fehlstellungen des Daumens (201) im Sattelgelenk (202), umfassend a) einen im wesentlichen formstabilen, U-förmig gebogenen Formkörper (10, 310, 410), der im angelegten Zustand zwischen Daumen (201) und Zeigefinger (203) auf die Hand (200) aufgesetzt ist und den Daumen (201) stützt und in einem Abstand zum Zeigefinger (203) fixiert, wobei der U-förmig gebogene Formkörper (10, 310, 410) einen ersten Endabschnitt (11, 311) und einen zweiten Endabschnitt (12, 312, 412) aufweist, und umfassend b) einen Gurt (20, 320, 420), der mit einem Gurtende (21, 321) am ersten Endabschnitt (11, 311) des Formkörpers (10, 310, 410) befestigt ist, wobei der zweite Endabschnitt (12, 312, 412) des Formkörpers (10, 310, 410) ein Führungselement (13, 313, 413) zum Führen des Gurts (20, 320, 420) aufweist, **dadurch gekennzeichnet, dass** der Gurt (20, 320, 420) vom ersten Endabschnitt (11, 311) aus gesehen im angelegten Zustand einmal so um das Handgelenk (204) geführt ist, dass er sich an einem Überlappungspunkt (22) selbst überlappt, wobei die beiden sich überlappenden Gurtabschnitte (20a, 20b, 320a, 320b) an dem Überlappungspunkt (22) nicht fest miteinander verbunden sind, und der Gurt (20, 320, 420) am Führungselement (13, 313, 413) des zweiten Endabschnitts (12, 312, 412) geführt wird und das Ende (23, 323) des Gurtes (20, 320, 420) am Gurt (20, 320, 420) reversibel befestigt werden kann.

2. Daumenorthese (100, 300, 400) nach Anspruch 1, wobei der zweite Endabschnitt (12, 312, 412) des Formkörpers (10, 310, 410) ein Umlenkelement (13, 313, 413) als Führungselement zum Umlenken Gurts (20, 320, 420) aufweist, wobei der Gurt (20, 320, 420) vom ersten Endabschnitt (11, 311) aus gesehen im angelegten Zustand einmal so um das Handgelenk (204) geführt ist, dass er sich an einem Kreuzungspunkt (22) als Überlappungspunkt selbst kreuzt, wobei die beiden sich kreuzenden Gurtabschnitte (20a, 20b, 320a, 320b) an dem Kreuzungspunkt (22) nicht fest miteinander verbunden sind, und der Gurt (20, 320, 420) am Umlenkelement (13, 313, 413) des zweiten Endabschnitts (12, 312, 412) umgelenkt wird.

3. Daumenorthese (100) nach einem der vorhergehenden Ansprüche, wobei das Umlenkelement (13) am zweiten Endabschnitt (12) des Formkörpers (10) als Einschnitt in den zweiten Endabschnitt (12) ausgebildet ist.

4. Daumenorthese (300) nach Anspruch 1 oder Anspruch 2, wobei das Führungselement (313) als Lasche zum Durchziehen des Gurts (320) ausgebildet ist.

5. Daumenorthese (400) nach Anspruch 1 oder Anspruch 2, wobei das Führungselement (413) den Gurt (420) über einen Dornverschluss(413b), über einen Klettverschluss oder über einen Haken/Ösen-Verschluss reversibel befestigt werden kann.

6. Daumenorthese (400) nach Anspruch 1 oder Anspruch 2 oder Anspruch 5, wobei das Führungselement (413) als Lochband (413a) und Fixierungselement (413b) ausgebildet ist.

7. Daumenorthese (100, 300, 400) nach einem der vorhergehenden Ansprüche, wobei der Überlappungspunkt (22), insbesondere Kreuzungspunkt (22) in einem definierten Überlappungsbereich (24, 324), insbesondere Kreuzungsbereich (24, 324) liegt.

8. Daumenorthese (100, 300, 400) nach einem der vorhergehenden Ansprüche, wobei der Überlappungsbereich (24, 324), insbesondere Kreuzungsbereich (24, 324) als Lasche (25, 325) an oder im Gurt (20, 320, 420) ausgebildet ist, durch die der Gurt (20, 320, 420) hindurchgezogen werden kann, wobei die Lasche (25, 325) in dem Bereich (20a, 320a, 420a) des Gurtes (20, 320, 420) angeordnet ist, der dem ersten Endabschnitt (11. 311) des Formkörpers (10, 310, 410) folgt.

9. Daumenorthese (100, 300, 400) nach Anspruch 8, wobei die Lasche (25, 325) einen Schlitz (26, 326) bildet, der breiter ist als die die Breite des Gurtes (20, 320, 420).

10. Daumenorthese (100, 300, 400) nach einem der vorhergehenden Ansprüche, wobei der Überlappungsbereich (24, 324), insbesondere Kreuzungsbereich (24, 324) als Klettverschluss ausgebildet ist.

11. Daumenorthese (100, 300, 400) nach einem der vorhergehenden Ansprüche, wobei das Ende (23, 323) des Gurtes (20, 320, 420) am Gurt (20, 320, 420) über einen Dornverschluss (27,28, 327, 328, 428), über einen Klettverschluss oder über einen Haken/Ösen-Verschluss reversibel befestigt werden kann.

12. Daumenorthese (100, 300, 400) nach einem der vorhergehenden Ansprüche, wobei der Formkörper (10, 310, 410) und der Gurt (20, 320, 420) einstückig ausgebildet sind.

13. Daumenorthese (100, 300, 400) nach einem der vorhergehenden Ansprüche, wobei der Formkörper (10, 310, 410) sattelförmig ist und Luftlöcher (14) aufweist und wobei der Formkörper (10, 310, 410) aus einem anformbaren Material besteht.

14. Daumenorthese (100, 300, 400) nach einem der vorhergehenden Ansprüche, wobei die Daumenorthese beim Tragen an einer Hand den Daumen in die Ebene der Handfläche zieht.

## Claims

1. Thumb orthosis (100, 300, 400), in particular for correcting malpositions of the thumb (201) in the carpometacarpal joint (202), comprising a) a substantially dimensionally stable molded body (10, 310, 410) bent in a U-shape, which in the applied state is fitted on the hand (200) between the thumb (201) and the index finger (203) and supports the thumb (201) and fixes it at a distance from the index finger (203), wherein the molded body (10, 310, 410) bent in a U-shape has a first end portion (11, 311) and a second end portion (12, 312, 412), and comprising b) a strap (20, 320, 420) which is fastened by one strap end (21, 321) to the first end portion (11, 311) of the molded body (10, 310, 410), wherein the second end portion (12, 312, 412) of the molded body (10, 310, 410) has a guiding element (13, 313, 413) for guiding the strap (20, 320, 420), **characterized in that,** as viewed from the first end portion (11, 311), in the applied state the strap (20, 320, 420) is guided once around the wrist (204) such that it overlaps itself at an overlap point (22), wherein the two mutually overlapping strap portions (20a, 20b, 320a, 320b) are not rigidly connected to one another at the overlap point (22), and the strap (20, 320, 420) is guided on the guiding element (13, 313, 413) of the second end portion (12, 312, 412), and the end (23, 323) of the strap (20, 320, 420) can be reversibly fastened to the strap (20, 320, 420).

2. Thumb orthosis (100, 300, 400) according to claim 1, wherein the second end portion (12, 312, 412) of the molded body (10, 310, 410) has a turning element (13, 313, 413) as a guiding element for turning the strap (20, 320, 420), wherein, as viewed from the first end portion (11, 311), in the applied state the strap (20, 320, 420) is guided once around the wrist (204) such that it intersects with itself at an intersection point (22) as an overlap point, wherein the two intersecting strap portions (20a, 20b, 320a, 320b) are not rigidly connected to one another at the intersection point (22), and the strap (20, 320, 420) is turned at the turning element (13, 313, 413) of the second end portion (12, 312, 412).

3. Thumb orthosis (100) according to one of the preceding claims, wherein the turning element (13) on the second end portion (12) of the molded body (10) is formed as a notch in the second end portion (12).

4. Thumb orthosis (300) according to claim 1 or claim 2, wherein the guiding element (313) is formed as a loop for pulling the strap (320) through.

5. Thumb orthosis (400) according to claim 1 or claim 2, wherein the guiding element (413) can be reversibly fasten the strap (420) via a pin closure (413b), via a hook and loop closure, or via a hook/eye closure.

6. Thumb orthosis (400) according to claim 1 or claim 2 or claim 5, wherein the guiding element (413) is formed as a perforated strap (413a) and fixing element (413b).

7. Thumb orthosis (100, 300, 400) according to any one of the preceding claims, wherein the overlap point (22), in particular intersection point (22), is located in a defined overlap region (24, 324), in particular intersection region (24, 324).

8. Thumb orthosis (100, 300, 400) according to any one of the preceding claims, wherein the overlap region (24, 324), in particular intersection region (24, 324), is formed as a loop (25, 325) on or in the strap (20, 320, 420), through which the strap (20, 320, 420) can be pulled, wherein the loop (25, 325) is arranged in the region (20a, 320a, 420a) of the strap (20, 320, 420) which follows the first end portion (11, 311) of the molded body (10, 310, 410).

9. Thumb orthosis (100, 300, 400) according to claim 8, wherein the loop (25, 325) forms a slot (26, 326) that is wider than the width of the strap (20, 320, 420).

10. Thumb orthosis (100, 300, 400) according to any one of the preceding claims, wherein the overlap region (24, 324), in particular intersection region (24, 324), is formed as a hook-and-loop closure.

11. Thumb orthosis (100, 300, 400) according to any one of the preceding claims, wherein the end (23, 323) of the strap (20, 320, 420) can be reversibly fastened to the strap (20, 320, 420) via a pin closure (27, 28, 327, 328, 428), via a hook-and-loop colsure, or via a hook/eye closure.

12. Thumb orthosis (100, 300, 400) according to any one of the preceding claims, wherein the molded body (10, 310, 410) and the strap (20, 320, 420) are integrally formed.

13. Thumb orthosis (100, 300, 400) according to any one of the preceding claims, wherein the molded body (10, 310, 410) is saddle-shaped and has air holes (14), and wherein the molded body (10, 310, 410) consists of a shapeable material.

14. Thumb orthosis (100, 300, 400) according to any one of the preceding claims, wherein the thumb orthosis pulls the thumb into the plane of the palm when worn on a hand.

## Revendications

1. Une orthèse de pouce (100, 300, 400), en particulier pour corriger des malpositions du pouce (201) dans l'articulation carpométacarpienne (202), comprenant a) un corps moulé (10, 310, 410) substantiellement indéformable courbé en forme de U, qui à l'état appliqué est raccordé sur la main (200) entre le pouce (201) et l'index (203) et soutient le pouce (201) et le fixe à distance de l'index (203), dans lequel le corps moulé (10, 310, 410) courbé en forme de U a une première partie d'extrémité (11, 311) et une deuxième partie d'extrémité (12, 312, 412), et comprenant b) une sangle (20, 320, 420) qui est fixée par une extrémité de sangle (21, 321) à la première partie d'extrémité (11, 311) du corps moulé (10, 310, 410), dans lequel la deuxième partie d'extrémité (12, 312, 412) du corps moulé (10, 310, 410) a un élément de guidage (13, 313, 413) pour guider la sangle (20, 320, 420), **caractérisé en ce que,** vu de la première partie d'extrémité (11, 311), à l'état appliqué, la sangle (20, 320, 420) est guidée une fois autour du poignet (204) de telle sorte qu'elle se chevauche elle-même à un point de chevauchement (22), dans lequel les deux parties de sangle (20a, 20b, 320a, 320b) qui se chevauchent mutuellement ne sont pas rigidement connectées l'une à l'autre au niveau du point de chevauchement (22), et la sangle (20, 320, 420) est guidée sur l'élément de guidage (13, 313, 413) de la deuxième partie d'extrémité (12, 312, 412), et l'extrémité (23, 323) de la sangle (20, 320, 420) peut être fixée de manière réversible à la sangle (20, 320, 420).

2. L'orthèse de pouce (100, 300, 400) selon la revendication 1, dans laquelle la deuxième partie d'extrémité (12, 312, 412) du corps moulé (10, 310, 410) a un élément de tournage (13, 313, 413) comme élément de guidage pour tourner la sangle (20, 320, 420), dans lequel, vu de la première partie d'extrémité (11, 311), à l'état appliqué, la sangle (20, 320, 420) est guidée une fois autour du poignet (204) de telle sorte qu'elle se croise avec elle-même à un point d'intersection (22) en tant que point de chevauchement, dans lequel les deux parties de sangle qui se croisent (20a, 20b, 320a, 320b) ne sont pas rigidement connectées l'une à l'autre au niveau du point d'intersection (22), et la sangle (20, 320, 420) est tournée au niveau de l'élément de tournage (13, 313, 413) de la deuxième partie d'extrémité (12, 312, 412).

3. L'orthèse de pouce (100) selon l'une des revendications précédentes, dans laquelle l'élément de tournage (13) sur la deuxième partie d'extrémité (12) du corps moulé (10) est formé comme une encoche dans la deuxième partie d'extrémité (12).

4. L'orthèse de pouce (300) selon la revendication 1 ou la revendication 2, dans laquelle l'élément de guidage (313) est formé comme une boucle pour tirer la sangle (320) à travers.

5. L'orthèse de pouce (400) selon la revendication 1 ou la revendication 2, dans laquelle l'élément de guidage (413) peut être fixé de manière réversible à la sangle (420) via une fermeture à broche (413b), via une fermeture à crochet et boucle, ou via une fermeture à crochet et oeillet.

6. L'orthèse de pouce (400) selon la revendication 1 ou la revendication 2 ou la revendication 5, dans laquelle l'élément de guidage (413) est formé d'une sangle perforée (413a) et d'un élément de fixation (413b).

7. L'orthèse du pouce (100, 300, 400) selon l'une quelconque des revendications précédentes, dans laquelle le point de chevauchement (22), en particulier le point d'intersection (22), est situé dans une région de chevauchement définie (24, 324), en particulier la région d'intersection (24, 324).

8. L'orthèse de pouce (100, 300, 400) selon l'une quelconque des revendications précédentes, dans laquelle la région de chevauchement (24, 324), en particulier la région d'intersection (24, 324), est formée comme une boucle (25, 325) sur ou dans la sangle (20, 320, 420), à travers laquelle la sangle (20, 320, 420) peut être tirée, dans laquelle la boucle (25, 325) est disposée dans la région (20a, 320a, 420a) de la sangle (20, 320, 420) qui suit la première partie d'extrémité (11, 311) du corps moulé (10, 310, 410).

9. L'orthèse de pouce (100, 300, 400) selon la revendication 8, dans laquelle la boucle (25, 325) forme une fente (26, 326) plus large que la largeur de la sangle (20, 320, 420).

10. L'orthèse de pouce (100, 300, 400) selon l'une quelconque des revendications précédentes, dans laquelle la région de chevauchement (24, 324), en particulier la région d'intersection (24, 324), est formée comme une fermeture à crochet et boucle.

11. L'orthèse pouce (100, 300, 400) selon l'une quelconque des revendications précédentes, dans laquelle l'extrémité (23, 323) de la sangle (20, 320, 420) peut être fixée de manière réversible à la sangle (20, 320, 420) par une fermeture à broches (27, 28, 327, 328, 428), par une fermeture à crochet et boucle, ou par une fermeture à crochet et à oeillet.

12. L'orthèse de pouce (100, 300, 400) selon l'une quelconque des revendications précédentes, dans laquelle le corps moulé (10, 310, 410) et la sangle (20, 320, 420) sont formés intégralement.

13. L'orthèse de pouce (100, 300, 400) selon l'une quelconque des revendications précédentes, dans laquelle le corps moulé (10, 310, 410) est en forme de selle et a des trous d'air (14), et dans laquelle le corps moulé (10, 310, 410) est constitué d'un matériau modelable.

14. L'orthèse de pouce (100, 300, 400) selon l'une quelconque des revendications précédentes, dans laquelle l'orthèse de pouce tire le pouce dans le plan de la paume lorsqu'elle est portée sur une main.
